# EUROPEAN PATENT APPLICATION

(11) **EP 1 321 472 A2**
(43) Date of publication of application: **25.06.2003**
(21) Application number: 01972392.3
(22) Date of filing: 05.09.2001
(51) Int. Cl.: C07K 14/37, A01N 63/04

(54) **ELICITOR OF FUNGAL ORIGIN AND METHOD FOR ITS PREPARATION**

(30) Priority: 19.09.2000 CO 7095900
(71) Applicant: Centro International de Fisica CIF, Bogota, D.C. A.A. Bogota (CO)
(72) Inventor: GUARDIOLA PERILLA, Marta, L., Cto. Int. de Fisica, Bogota, D.C. A.A. Bogota (CO)
(74) Representative: King, Elizabeth Caroline
(86) International application number: IB0101850
(87) International publication number: WO02024869

(57) **Abstract**

The present invention discloses a process for preparing an elicitor from *Fusarium oxysporum* and the product obtained by means of said process, i.e., an elicitor of systemic resistance in plants. The process is based upon successive extraction of cytoplasmatic fractions from the membrane and the wall of fungus *F. oxysporum,* mixture which constitutes the mentioned product.

## Description

The present invention is related to the agricultural field. Particularly, the subject matter of the present invention provides a process for preparing a fungal elicitor and a product obtained by means of said process, i.e., an elicitor inductor of systemic resistance in plants, as an alternative to fungicides.

### BACKGROUND OF THE INVENTION

In the agricultural field, the diseases and pests control in plant species, as distinguished from the animal species, was concentrated in the use of synthetic organic pesticides during the majority of XX century. This fact was due to the growing demand of agricultural products for the industry and human consumption, which force the establishment of an extensive agriculture of monocultures with selected seeds, which in its turn requires highly efficient control of pests. DDT, organophosphorus compounds and carbamates were the answer of the chemical industry to this demand and they were established as fundamental part of worlds agriculture. The use of pesticides for the control of plant diseases and other pests has increased at a constant rate of approximately 14% per year since the middle of 50's. The world consumption of pesticide in 1977 was about 2.3 billions of kilograms. Nowadays, in 2000 year, it is calculated in more than 3 billions of kilograms.

During 60's and 70's, the development of new diseases and pests, species extinction, appearance of rare human diseases, residuality diagnostics in waters and soils, among others, awared the public abut the harmful effects of using of these synthetic products. The 80's decade marks the beginning of a worldwide "ecological conscience", which implies the need of taking into account natural variability as a basic tool for the continuity of the species, including man, on the planet. Therefore, presently the demand is directed toward biological based technologies for controlling diseases, said technologies being cleaner for the environment including human beings.

The invention, subject matter of the present application, is directed to provide a clean alternative for controlling diseases in plants.

### Plant Defense Mechanisms and Systemic Induced Resistance

The plants do not count with a defense system mediated by antibodies as the animals. However, they have constitutive strategies which act as fungistatic or bacteriostatic, delaying the penetration rate of the pathogen. Said constitutive strategies are preformed barriers and a series of enzymes and toxins which affect the pathogen, inhibiting their growth and/or discouraging the attack. Furthermore, the plants rely on active defense mechanisms comprising a complex system of inducible responses, which are manifested through the synthesis of proteins, carbohydrates and secondary metabolites, with activities similar to those described above.

The defense compounds of the plants can be classified according to their size and mode of action (Kuc, J. 1995. Systemic Induced Resistance as part of integrated plant disease control. In: "Environmental biotic factors in integrated plant disease control" (M. Manka, Ed.). The Polish Phytopathological Society, Poznan. 129-136; Stermer, B.A., 1995, Molecular regulation of systemic induced resistance. In: Induced resistance to Disease in Plants, pp 111-140 (De. Hammershmidt, R. y Kuc, J.), Dodrecht Kluwer). The high molecular weight compounds include those functioning as physicochemical barriers, e.g., cutin, suberin, lignin, callose, glycoproteins enriched with hydroxyproline and glycine, phenols crossed-over with wall polysaccharides and pigments. A second group of defense compounds of high molecular weight includes enzymes which can act directly over the pathogen such as chitinases, β-1,3 glucanase, proteases and ribonucleases, and other enzymes that can generate reactive molecules by hydrolithic action, such as β and α glycosidases and peroxidases (Bolwell, P.G., 1988 Synthesis of cell wall components: aspects of control. Phytochemistry. Vol 27. 5: 1235-1253; Mackenbrock, U., Gunia, W., Barz, W., 1933. Accumulation and metabolism of medacarpin and maackiain malonyglucosides in elicited chick pea *(Cicer prietinum L*.) Cell suspension cultures. Journal Plant Physiol. 142: 385-391).

The second group of defense compounds corresponds to substances with antimicrobial activity and low molecular weight, which can be considered analogous to pesticides. These include compounds formed in the plant prior to infection (e.g. tannins, sucrose esters and steroids), compounds released or modified as a result of the infection or damage (tannins, glycosides and phenols), compounds (phytoalexins) locally synthesized around the infection or stress site (Kuc, 1995. Phytoalexins, stress metabolism and disease resistance in plants. Ann. Review of Phytopathology. 33: 275-297).

The defense mechanisms of plants can be activated in a natural way during contact with the invader. The outcome of which can be beneficial, neutral or detrimental (infection) for the plant. On the other hand, the same defense mechanisms can be induced in the plants by means of chemical substances (salicylic acid analogous (BION)), microorganism fractions (virus, bacteria or fungi), attenuated pathogens, extracts from plants and metabolites thereof, among others. These substances inducing the process of vegetable defense are known as elicitors (Ebel. J. & Cosio, E.G., 1994, Elicitors of plant defense response. Int. Rev. Cytol. 148: 1-36) and the resistance conferred in this way has been defined as induced resistance (Sticher, L., Mauch-Mani, b., Metraux, J.P. 1997. Systemic Acquired Resistance. Ann. Rev.' Phytopathol. 35: 235-70). The type of induced resistance which is not located but with a systemic character (expressed in all tissues of the plant) is called "Systemic Induced Resistance" or SIR.

In contrast to animal systems, it has been demonstrated that SIR has a broad spectrum since it induces protection against multiple agents causing diseases, including virus (Chester, K.S., 1933. The problem of acquired physiological immunity in plants. Q. Rev. Biol. 8: 275-324; Kuc, J.1982. Induced Immunity to plant disease. BioScience 32:854-860; Kur, J. 1933. Induced Systemic Resistance - a non pesticide technology for disease control in plants. New directions in pesticides resarch, development, management, and policy. Proceeding of the fourth national conference on pesticides; Ryals, J., Uknes, S., Ward, E. 1994. Systemic Acquired Resistance. Plant Physiology 104: 1109-1112).

### The Elicitors

The elicitors of resistance from natural origin, from plants, fungi, bacteria and virus pathogens as well as non pathogens, alive or fractions thereof, are known in the state of the art, as it can be observed, for example, in document WO 00/20616; or in document of Rohwer et al. (Rohwer, F., Fritzemeier, K.H., Scheel, D., and Halbrock, K. 1987. Biochemical reactions of different tissues of potato (*Solanum tuberosum*) to zoospores or elicitors from *Phytophtora infestans.* Planta 170: 556-561) which describes an elicitor prepared from the culture media of *Phytophtora infestans;* and in the document of Reglinski et al. (Reglinski, T., Newton, A.C., and Lyon, G.D. 1990, Elicitor-active yeast cell wall components as a novel crop protectant. BCPC, Mono No. 45 Organic and low input agriculture. 231 - 243) describing an elicitor prepared from yeast (*Saccaromyces cerevisiae*). In the same way, chemically synthesized elicitors are known such as BION, an analogous from salicylic acid produced by NOVARTIS company. These elicitors are distinguished by being isolated molecules and they are physicochemical characterized in the case of those from natural origin, or chemically synthesized.

The fundamental action mechanism of the elicitors from the state of the art are based in the stimulus of a particular metabolic route which depends upon the characteristics of the selected elicitor. In other words, the action mechanisms of the elicitors known in the state of the art are ruled by the vertical resistance theory (gene by gene, Flor, H., 1971, Models of host pathogen interaction in flax rust: its genetics and other simplifications. Ann. Rev. Phytopathol, 9: 275-296), which states that to each gene (R-dominant) for the host resistance (the plant) corresponds an avirulence gene of the pathogen (P-dominant). This theory assumes that the gene controlling the recognition of a signal molecule produced by the pathogen is a resistance gene and the gene of the pathogen controlling the synthesis of the recognized substance (microorganism's product) has been converted in an avirulence gene. In this case, the natural or synthetic elicitor is a product homologous of the avirulence gene product.

Even though, the elicitors based in the vertical resistance mechanism provide an alternative for the use of pesticides in the agricultural field, there is a need of supplying new improved elicitors which provide a systemic induced resistance (SRI) in plants.

### Fusarium oxysporum fungus

The resistance elicitors from natural origin are produced from plants, fungi, bacteria, and virus pathogens as well as non pathogens, alive or fractions thereof. A fungus of particular relevance is *Fusarium oxysporum* which, up to the present invention, has not been used in the manufacture of an elicitor. *Fusarium oxysporum* is a cosmopolitan fungus which causes the vascular wilt of manifold species of angiosperms and gymnosperms, among coffee, banana, carnation, tomato, cotton, palm nuts, naranjilla and many others.

Vascular wilt in tomato by *Fusarium* was reported by the first time in 1986, and since then, the interest in this disease has continued due to its worldwide distribution. In said disease, the pathogen establishes in the vascular system and with odd exceptions, the pathogen does not leave it but up to the host's death. The pathogen generally penetrates through the cell walls of the root tip, although, it can also colonize through the hypocotyl and cotyledons. It moves inter or intracellular through the parenchymatous tissues, invading them and living within the xylem vessels as a saprophyte. The effect in the host is the occlusion of the water transportation system (Beckman 1987, The nature of wilt diseases in plants. APS Press USA. pp.17-21).

*Fusarium oxysporum* is a fungus that belongs to the genus deuteromycotina, which includes species whose sexual state (teleomorph) is unknown. *Fusarium* species have a common characteristic: the production of macroconidias, which can be multinucleate and with different geometrical forms, sizes, colors, and architecture. These characteristics as a whole constitute the basis for subdividing the genus in species.

Nowadays, *Fusarium oxysporum's* control in different crops ranges from the intensive use of chemical pesticides of different origins to soil vaporization. However, in view of the infection form, i.e. starting from the root, the chemical control has not been very successful and the vaporization is highly expensive and it just exterminates the fungus up to 20 centimeters of depth, covering only a period of culture free from the disease.

*Fusarium oxysporum* is an unique tool for the production of elicitors in view of its broad spectrum of hosts, its pathogenic mechanism and its omnipresence.

### ABSTRACT OF THE DISCLOSURE

The first aspect of the invention, is provided by a process for preparing an elicitor from the fungus *Fusarium oxysporum*.

A second aspect of the invention, is provided by a *Fusarium oxysporum* elicitor, obtained from the above cited process, which stimulates "systemic induced resistance" in the plants over which it is applied.

Moreover, a third aspect of the invention, is provided by an agricultural composition comprising *Fusarium oxysporum* elicitor together with a suitable agricultural carrier.

The subject matter of the present invention, which comprises the process for preparing the elicitor, the obtained elicitor prepared from the mycelium of *Fusarium oxysporum* fungus and the agricultural composition comprising the said elicitor, has as a main purpose the provision of a biotechnological alternative to the use of chemical pesticides, stimulating in the plants their natural defense mechanisms, in order to control the effect of fungal, bacterial and viral pathogens and in some cases, the pests.

### BRIEF DESCRIPTION OF THE DRAWINGS

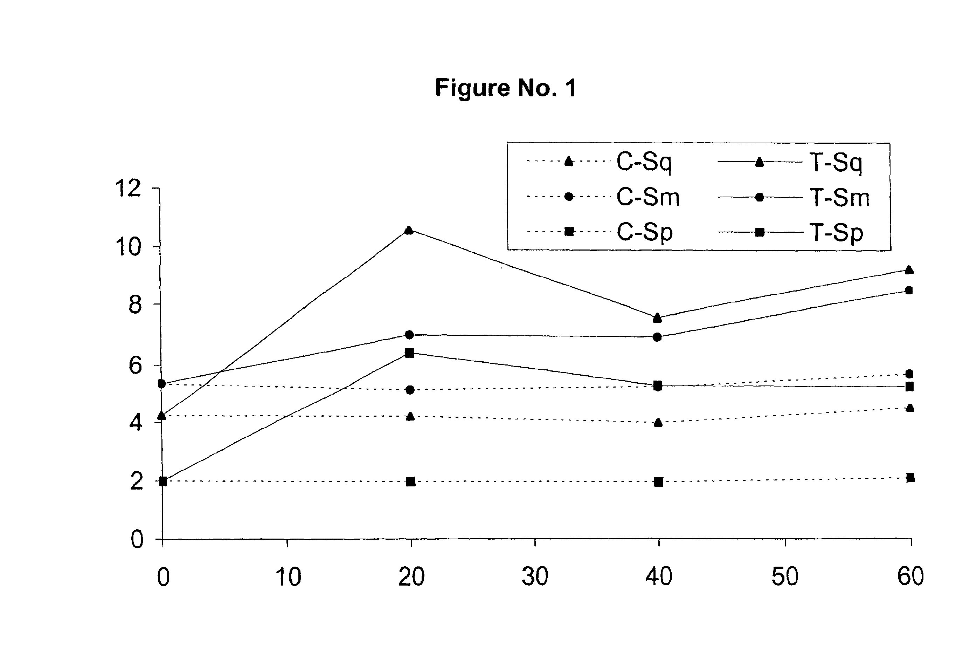

Figure No. 1. Examination of the callose contents in leaves from plants (*Solanum quitoense*, *Solanum marginatum* and *Solanum pseudolulo*) treated with the elicitor of the present invention. On said figure, mg callose/g material are in axis Y and, the days after inoculation are established in axis X. The conventions are: C-Sq = *Solanum quitoense* Control, T-Sq = *Solanum quitoense* Treated, C-Sm = *Solanum marginatum* Control, T-Sm = *Solanum marginatum* Treated, C-Sp = *Solanum pseudolulo* Control, T-Sp = *Solanum pseudolulo* Treated.

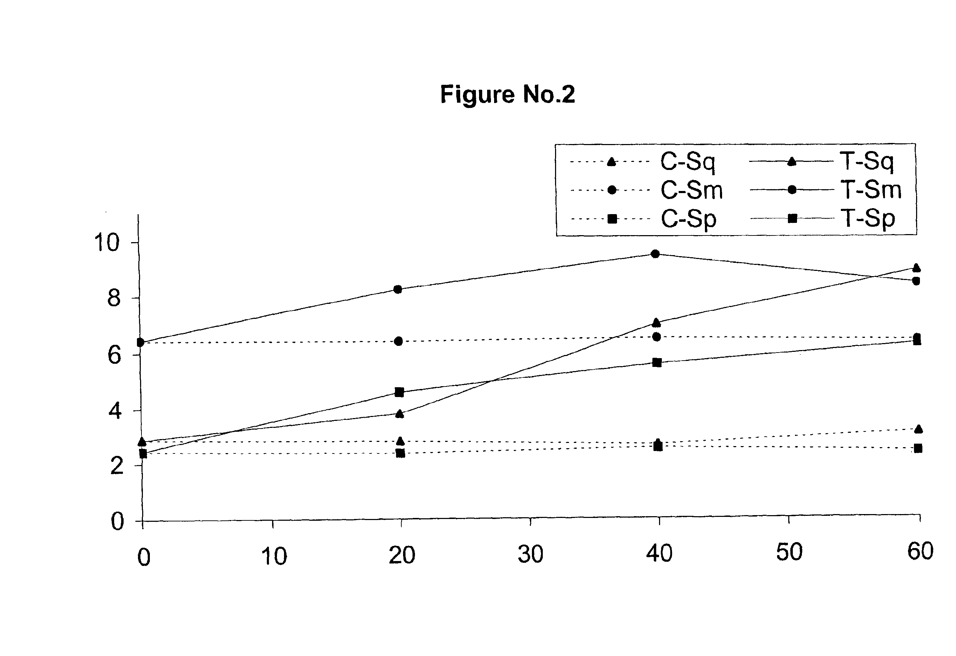

Figure No. 2. Examination of the callose contents in roots from plants *(Solanum quitoense, Solanum marginatum* and *Solanum pseudolulo)* treated with the elicitor of the present invention. On said figure, mg callose/g material are in axis Y and, the days after inoculation are in axis X. The conventions are: C-Sq = *Solanum quitoense* Control, T-Sq = *Solanum guitoense* Treated, C-Sm = *Solanum marginatum* Control, T-Sm = *Solanum marginatum* Treated, C-Sp = *Solanum pseudolulo* Control, T-Sp = *Solanum pseudolulo* Treated.

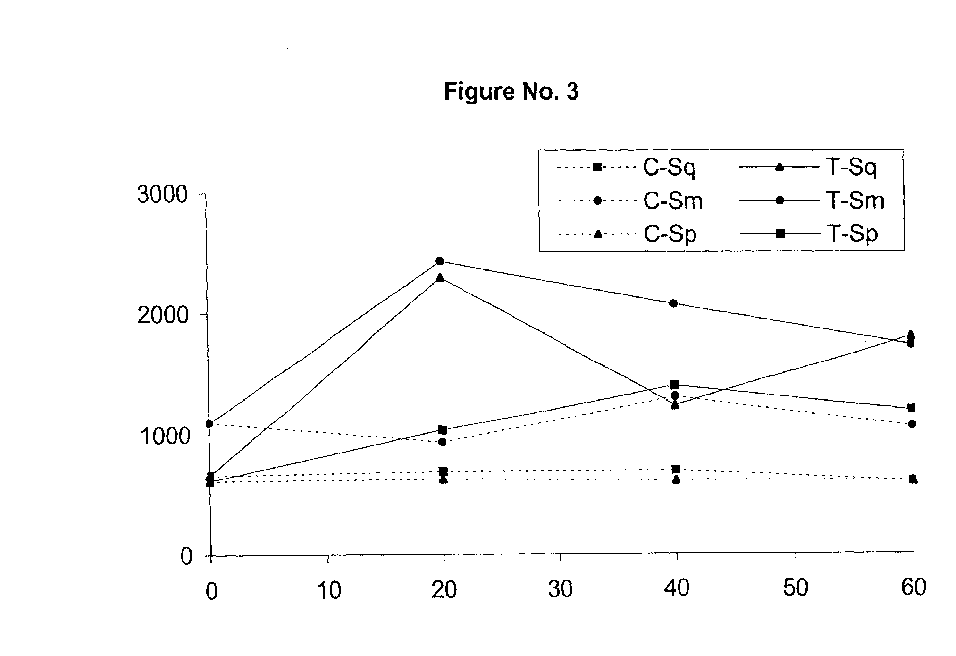

Figure No. 3. Examination of β-1,3 glucanase activity in leaves from plants *(Solanum quitoense, Solanum marginatum* and *Solanum pseudolulo*) treated with the elicitor of the present invention. On said figure, the enzymatic activity is in axis Y and the days after inoculation are established in axis X. The conventions are: C-Sq = *Solanum quitoense* Control, T-Sq = *Solanum quitoense* Treated, C-Sm = *Solanum marginatum* Control, T-Sm = *Solanum marginatum* Treated, C-Sp = *Solanum* *pseudolulo* Control, T-Sp = *Solanum pseudolulo* Treated. An unit of enzymatic activity is equal to the quantity of enzyme that produces 1 µmol of reducing sugars from oarweed laminarin (*Laminaria digitata -* SIGMA) by minute, under the assay conditions.

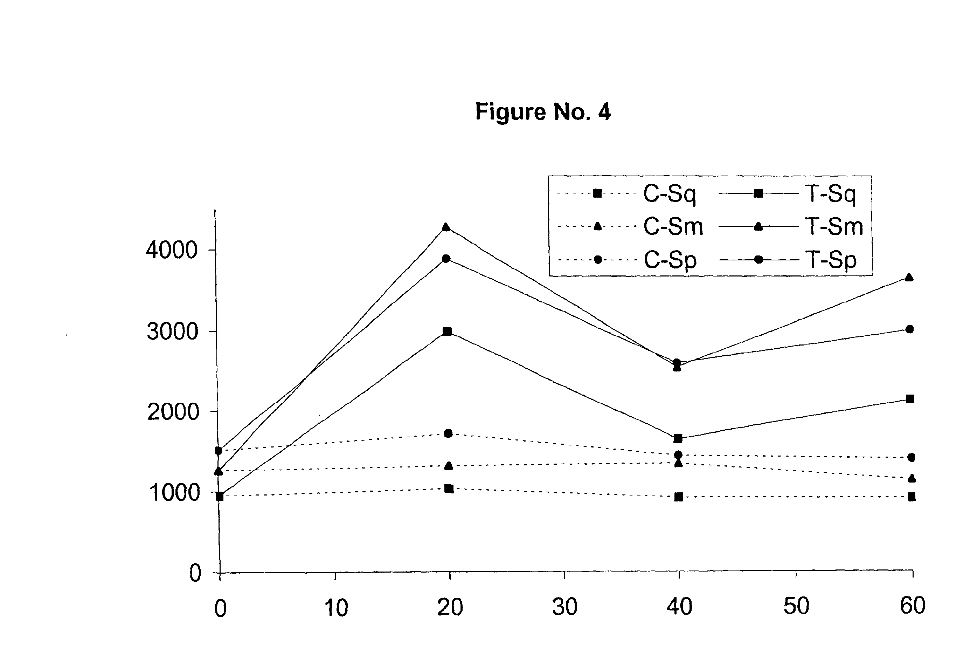

Figure No. 4. Examination of β-1,3 glucanase activity in roots from plants (*Solanum quitoense, Solanum marginatum* and *Solanum pseudolulo)* treated with the elicitor of the present invention. On said figure, the enzymatic activity is in axis Y and, the days after inoculation are established in axis X. The conventions are: C-Sq = *Solanum quitoense* Control, T-Sq = *Solanum quitoense* Treated, C-Sm = *Solanum marginatum* Control, T-Sm = *Solanum marginatum* Treated, C-Sp = *Solanum pseudolulo* Control, T-Sp = *Solanum pseudolulo* Treated. An unit of enzymatic activity is equal to the quantity of enzyme that produces 1 µmol of reducing sugars from oarweed laminarin (*Laminaria digitata -* SIGMA) by minute, under the assay conditions.

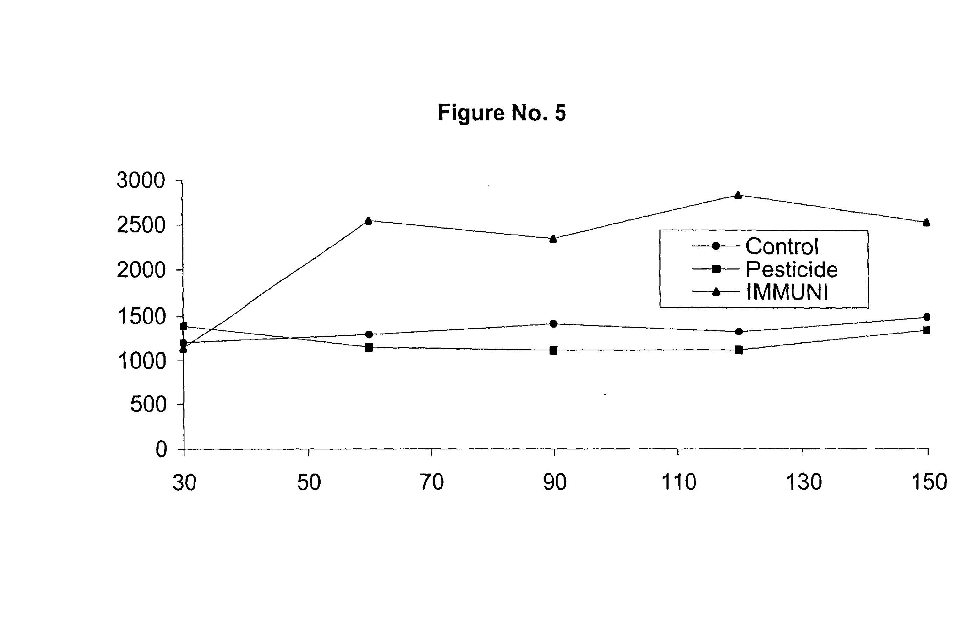

Figure No. 5. Examination of β-1,3 glucanase activity in leaves from potato plants (*Solanum tuberosum*) treated with the elicitor of the present invention, in comparison to leaves of potato plants over which pesticides were applied, and leaves of potato plants without treatment (control), along a harvest time. On said figure, the enzymatic activity is in axis Y and, the days of culture are established in axis X. An unit of enzymatic activity is equal to the quantity of enzyme that produces 1 µmol of reducing sugars from oarweed laminarin (*Laminaria digitata -* SIGMA) by minute, under the assay conditions.

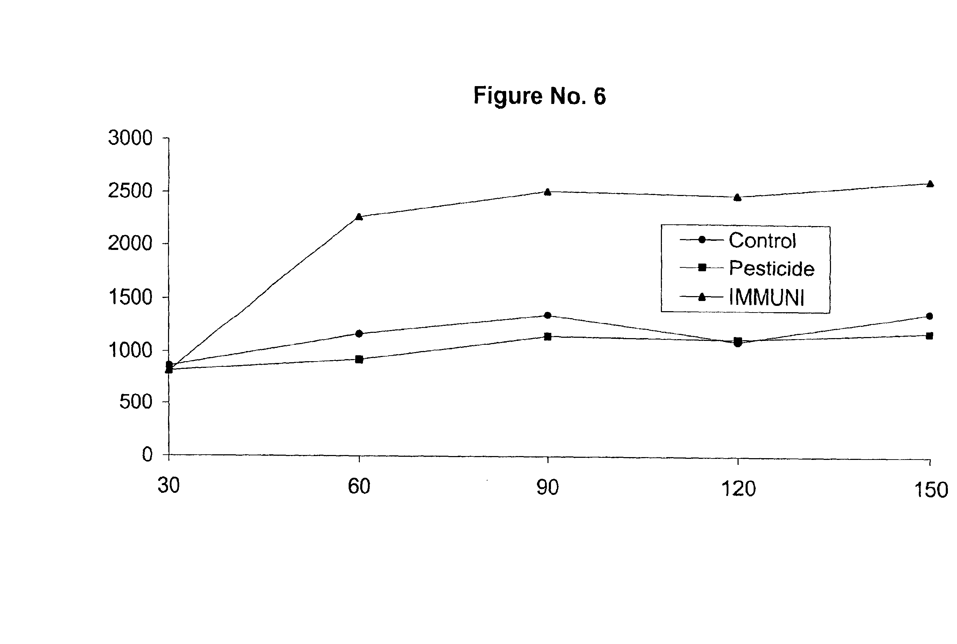

Figure No. 6. Examination of β-1,3 glucanase activity in roots from potato plants (*Solanum tuberosum*) treated with the elicitor of the present invention, in comparison to roots - of potato plants over which pesticides were applied, and roots of potato plants without treatment (control), along a harvest time. On said figure, the enzymatic activity is in axis Y and, the days of culture are established in axis X. An unit of enzymatic activity is equal to the quantity of enzyme that produces 1 µmol of reducing sugars from oarweed laminarin (*Laminaria digitata -* SIGMA) by minute, under the assay conditions.

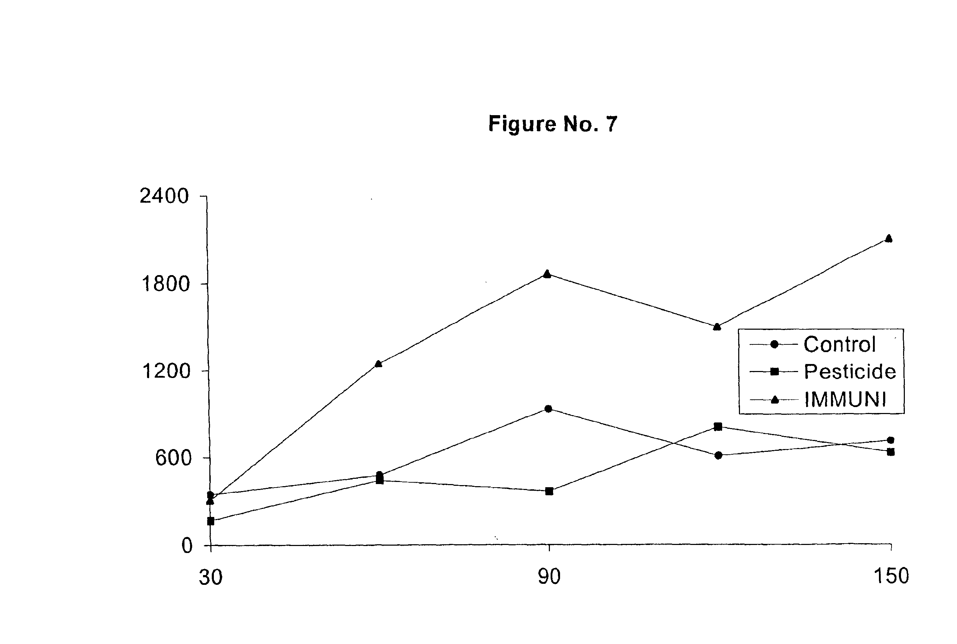

Figure No. 7. Examination of peroxidase activity in leaves from potato plants *(Solanum tuberosum*) treated with the elicitor of the present invention, in comparison to leaves of potato plants over which pesticides were applied, and leaves of potato plants without treatment (control), along a harvest time. On said figure, the enzymatic activity is in axis Y and, the days of culture are established in axis X. An unit of peroxidase activity is defined as the quantity of enzyme which oxidates 1 µmol of guaiacol/min, under the assay conditions.

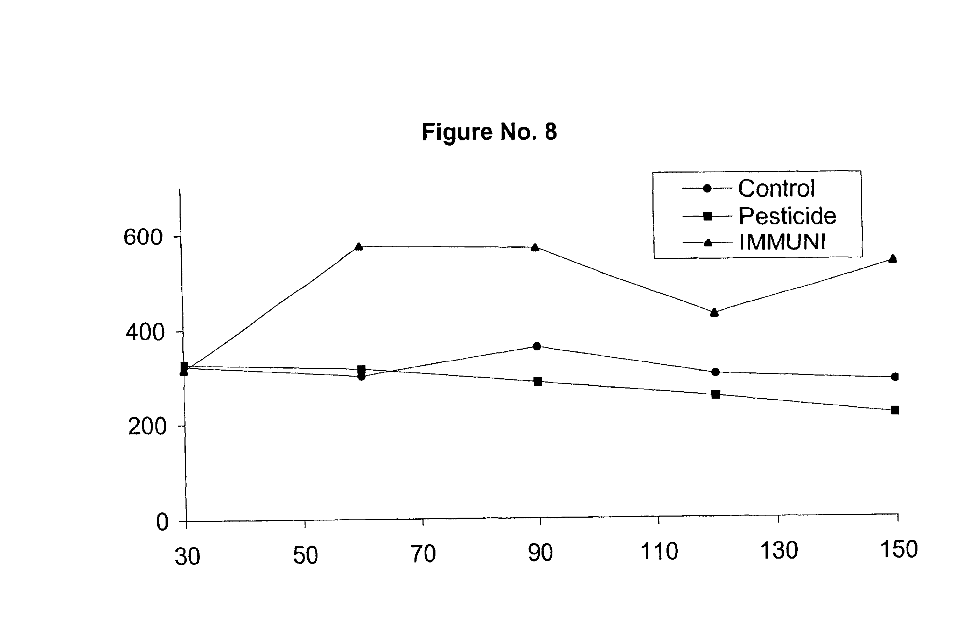

Figure No. 8. Examination of peroxidase activity in roots from potato plants (*Solanum tuberosum)* treated with the elicitor of the present invention, in comparison to roots of potato plants over which pesticides were applied, and roots of potato plants without treatment (control), along a harvest time. On said figure, the enzymatic activity is in axis Y and, the days of culture are established in axis X. An unit of peroxidase activity is defined as the quantity of enzyme which oxidates 1 µmol of guaiacol/min, under the assay conditions.

### DETAILED SPECIFICATION OF THE INVENTION

In a first embodiment, the present invention provides a process for preparing an elicitor obtained from *Fusarium oxysporum,* characterized by comprising the steps of:
(i) revirulentation *Fusarium oxysporum* in a host and cultivation of the said fungus;
(ii) sequentially extracting the fungus components through the steps:
   a) filtering and washing the fungal mycelium with distilled water or with aqueous solutions;
   b) breakage by freezing the isolated fungus between -40 °C and - 170 °C, followed by powdering;
   c) extracting the powder obtained in b) using a buffer in a pH range between 4 and 7, for its subsequent sonication by ultrasound, sterilization by heat and centrifugation of the resulting suspension; being the supernatant fraction A;
   d) extracting the centrifugation residual of the suspension using an alcoholic solvent and subsequent storage at a temperature between 0 and 10 °C during a time between 20 and 200 hours, after that the alcoholic solvent is eliminated, and the residue is resuspended in a basic solution between pH 12 and 14 with incubation at temperature between 70 and 100 °C for at least 0.5 hours. The obtained mixture is adjusted to pH between 5,0 and 7,0 with an acid solution and then, centrifuged, being the supernatant fraction B;
(iii) mixing fractions A and B obtained in step ii), being fraction A between 10 and 50% of the mixture and fraction B between 90 and 50% of the mixture.

Particularly, revirulentation of the fungus, *Fusarium oxysporum,* can be carried out over naranjilla (*Solanum quitoense*), tomato (*Solanum esculentum*), and potato (*Solanum tuberosum*) plants or other host.

Particularly, the culture of the fungus *Fusarium oxysporum* can be made over solid Czapeck media or its equivalents (e.g., PDA (potato dextrose agar), Malt Extract, Sabouraud, or Martin Agar).

Particularly, breakage by freezing of the fungus *Fusarium oxysporum* is carried out using liquid nitrogen or other liquefied gas at temperatures between -40 °C and - 170 °C.

Particularly, the buffer used for extracting the broken (powdered) fungus can be a phosphate buffer, citrate or ascorbate.

Particularly, the sterilization by heat carried out in step (ii) is made in autoclave.

Particularly, the alcoholic solvent used in the extraction of the centrifugation residual can be ethanol.

Particularly, the basic solution used for resuspending the centrifugation residual can be NaOH between 0.01 and 2N and the acid solution can be HCl between 6 and 12 N.

Particularly, in the mixture of step (iii), fraction A is a 17% of the mixture and fraction B is a 83% of the mixture.

In a second embodiment, the present invention supplies the elicitor obtained by means of the above described process, which comprises a mixture of a fraction A and a fraction B obtained from the fungus *Fusarium oxysporum* in a ratio 1:5, being fractions A and B corresponding to those obtained in the above described process.

Particularly, fraction A contains proteins, among which 70 to 95% are glycoproteins, from which 15 to 20% contain mannose and arabinose residues, and from 80% to 85% contain galactose. Said proteins have anionic characteristics and they are in a molecular weight range from 6.800 Da (1,122 x 10⁻²⁰ g) to 76.000 Da (1,254 x 10⁻¹⁹ g) .

Particularly, fraction B is composed by proteins and polysaccharides of the cell wall of *Fusarium oxysporum* fungus. 60% of the proteins are mannoproteins having anionic characteristics and they are in a molecular weight range from 10.000 Da (1,65 x 10⁻²⁰ g) to 65.000 Da (1,0725 x 10⁻¹⁹ g). The polysaccharides of said fraction B contain glucans (from 55 to 70% of the whole), of which from 15 to 25% have β-1,3 bonds, and N-acetyl glucosamine residues (from 0.1 to 0.9% of the whole), complex hemicelluloses of the xyloglucans type (from 8 to 13% of the whole) and reducing sugars.

Particularly, the elicitor of the present invention is characterized by a contents of reducing sugars and proteins in a ration of 3:1, respectively.

In a third embodiment, the present invention provides an agricultural composition characterized by comprising the *Fusarium oxysporum* elicitor of the present invention together with an acceptable agricultural carrier.

Particularly, the agricultural carrier should not contain ionic or non-ionic detergents.

Particularly, the agricultural carrier can be sodium carbonate with a concentration between 30 and 60 mM. Said sodium carbonate helps to the union between the elicitor of the present invention and the foliar surface.

Particularly, the agricultural composition of the present invention is characterized by being applied by spraying.

The elicitor of the present invention is characterized by accelerating the defense metabolism of the plants. Each one of the components of the elicitor of the present invention, i.e. fraction A and fraction B and the above mentioned individual components (glycoproteins, mannoproteins, hemicelluloses, reducing sugars, lipids, etc.), have the individual capability of inducing systemic resistance, however, the optimal effect of protection is obtained with the complete product. The biodegradability of the product is 12 hours at 18 °C in diluted form.

The elicitor of the present invention does not contaminate water or soils.

The elicitor of the present invention stimulates an increment of populations of beneficial rhizospherical microorganisms (*Trichoderma ssp*., *Gliocladium ssp.* and *Pseudomonas fluorescens*, among *others*).

### Effect of the elicitor of the present invention over the plants

The elicitor of the present invention prepared as it was mentioned above, has a stimulant effect of the defense mechanisms of the plants when it is applied over healthy plants. This inducing effect of Systemic Resistance is ruled by the horizontal resistance theory, which stipulates that the resistance of the plants depends upon the collective activity of the defense compounds, its appearance in suitable order and quantity depends upon a multigenic response, expressed with the simultaneous activation of several metabolic routes (Kuc, J. 1982. Induced Immunity to plant disease. BioScience 32:854-860).

The product, when applied to plants, stimulates among others:
■ Induction of enzymatic activity of proteins related with pathogenicity (β-1,3 glucanases, peroxidases and chitinases).
■ Increase of the total protein contents.
■ Increase of the polysaccharide contents.
■ Acceleration of the metabolic rate of the treated plants.

These metabolic changes are systemic (they occur along the whole plant, roots and leaves) and the duration of the response is from weeks to months with just one inoculation.

### Application of the elicitor of the present invention in the agriculture

The elicitor of the present invention is concentrated and therefore, it should be diluted 100 times for its application on the field. The final concentration of the elicitor of the present invention is expressed as reducing sugars (3.6 mg/ml) and total proteins (1.2 mg/ml).

The applications of the product in order to obtain optimal results of pathogen control, should be consecutive applications in youthful plants (two days after they germinated), 12 days post germination and each 30 days during the culture cycle. The volume of the applications is 3 ml of diluted liquid product. This is valid in the case of plants of transitory crops. The volume of application is the same for perennial, but the optimal concentration is the double. The application is made by spraying.

Under no circumstances, detergents of ionic or non-ionic character, which are found in the commercial adjuvants, should be used since they dilute the membrane receptors with protein character.

The preparation, characterization and determination of the elicitor activity of the present invention and the results obtained with its application on young plants, should be better understood through the description of the following examples, which illustrate the subject matter of the present invention, without limiting the scope of the claims.

### Example 1

### Preparation of the Fusarium oxysporum elicitor according to the present invention

### Revirulentation and culturing the fungus

The revirulentation of the fungus is made placing 20 microliters of a solution of conidias from *Fusarium oxysporum* (3 x 10⁴/ml) over naranjilla, tomato, potato plants or over other host cultured under complete sterility conditions (*in vitro*). Isolations of the fungus is done starting from the stems of these plants, which cultured on solid Czapeck media or its equivalents (p.ej., PDA (potato dextrose agar), Malt extract, Saboraud, or Martin agar) between 2 and 6 days in a growth room (temperature 24 ± 2 °C, photoperiod of 13 hours light).

Starting from these isolates, cultures on solid Czapeck media or its equivalents (e.g., PDA (potato dextrose agar), Malt extract, Sabouraud, or Martin agar) are established for 48 hours, under the same above describe conditions. Gathering of conidias is done by centrifugation. These are counted, and standard solutions of the fungus are established keeping the conidias on NaCl 0.02M at 4°C.

The fungus is massively propagated culturing the conidias in a bioreactor, on liquid Czapeck media or its equivalents (e.g., PDA (potato dextrose agar), Malt extract, Saboraud, or Martin agar) between 10 and 15 days in growth room (temperature 24 ± 2 °C, photoperiod of 13 hours light).

### Preparation of fractions A and B by means of consecutive extraction

The fungal mycelia produced in the bioreactor is vacuum filtered through a nylon membrane of 22 µm, washed with distilled water and broken by freezing with liquid nitrogen and homogenization in a blender.

The powder obtained this way is resuspended in a sodium phosphate buffer (between 0.8 and 0.05 M pH between 5 and 7) or its equivalents (e.g., citrate, ascorbate) in ratios 1:1 (weight/volume). The obtained suspension is submitted to sonication by ultrasound (intensity from 80 to 150 W) and sterilized in autoclave (from 20 to 30 min, 120 psi (0,8273 Pa)) so as to increase solubility, and thereafter, is centrifuged (from 5.000 r.p.m. (523,5 rad/s) and 10.000 r.p.m. (1047 rad/s) for 15 min).

The supernatant is kept at 4 °C. This supernatant corresponds to fraction A.

The centrifugation residue of fraction A is submitted to maceration with ethanol (95% - ratios of 1:2 weight volume) and submitted to incubation at 4 °C from 24 to 72 hours, in order to precipitate polysaccharides.

Ethanol is eliminated by vacuum filtration and the residue is resuspended in NaOH between 0.1 and 2N (ratios 1:3 weight volume). This mixture is submitted to incubation (92 °C from 0.5 to 4 hours). The complex polysaccharides are solubilized in sodium hydroxide at high temperatures.

The above mixture's pH is adjusted to 6.0 with HCl 12N and centrifuged (between 8.000 r.p.m. (837,8 rad/s) and 10.000 r.p.m. (1.256,6 rad/s) by 50 min). The supernatant of said centrifugation corresponds to fraction B.

### Mixture of fractions A and B

17% of fraction A is mixed with 83% of fraction B in order to obtain the *Fusarium oxysporum* elicitor according to the present invention.

### Example 2

### Characterization of the Fusarium oxysporum elicitor according to the present invention obtained in Example 1

Contents of total proteins and reducing sugars, present in fractions A and B of the *Fusarium oxysporum* elicitor, according to the present invention obtained in example 1, are quantified using the methodologies described by Bradford (Bradford, M., 1976. A rapid and sensitive method for the quantitation of micrograms quantities of protein using the principle of protein dye binding. Anal. Biochem. 72:248-254) and modified by Stoscheck (Stoscheck, C.M. 1990. Increased uniformity in the response of the Coomasie Blue G protein assay to different proteins. Analytical Biochemistry. 184: 111-116) for proteins, and the methodologies described by Somogyi (Somogyi, M. (1952). Notes on sugar determination. Journal of Biological Chemistry. 195: 19-23) and Nelson (Nelson, N. (1944) A photometric adaptation of the Somogyi method for the determination of sugars. Journal of Biological Chemistry. 153: 257-262) for reducing sugars.

Fraction A is composed by proteins, lipid and polysaccharides form the cytoplasm and from the membrane of the fungus. The protein components of this fraction have been partially characterized by affinity chromatography (Sepharose ConA), ion-exchange chromatography (Sephadex G-25) and gel filtration chromatography (seph). Said analysis of fraction A show that 90% of the proteins are glycoproteins, 17.5% of them contain mannose and arabinose residues, and the remainder (82.5%) contain galactose residues. Said glycoproteins have anionic characteristics and they are in molecular weight ranges from 6.800 Da (1,122 x 10⁻²⁰ g) to 76.000 Da (1,254 x 10⁻¹⁹ g).

Fraction B of *Fusarium oxysporum* mycelium prepared as it was describe in Example 1, is composed by proteins and polysaccharides of the cell wall of the fungus. The protein components and polysaccharides of this fraction B were partially characterized by affinity chromatography, by gel filtration chromatography and by spectrophotometric analysis. Said analysis of fraction B show that 60% of the proteins are mannoproteins having anionic characteristics and they are in a molecular weight range from 10.000 Da (1,65 x 10⁻²⁰ g) to 65.000 Da (1,0725 x 10⁻¹⁹ g). The polysaccharides of this fraction B contain glucans (64% of the whole), of which 23% have β-1,3 bonds, and N-acetyl glucosamine residues (0.5% of the whole), complex hemicelluloses of the xyloglucans type (11% of the whole) and reducing sugars.

The contents of reducing sugars and proteins of the elicitor of the present invention are found in a ratio which is always maintained in 3:1, respectively. Said ratio is a characteristic of the fungus strain and of the isolated fractions thereof, with the above described process.

### Example 3

### Agricultural composition comprising the elicitor of the present invention

The preparation of the agricultural composition comprising the elicitor of the present invention is described afterwards. The elicitor of the present invention prepared according to the process described in Example 1 is mixed with sodium carbonate in a concentration from 30 to 60 mM, so as to help the union between the elicitor of the present invention and the foliar surface. In said agricultural composition, the use of detergents of ionic or non-ionic character, which are found in the commercial adjuvants, was avoided since these dilute the membrane receptors of proteinaceous character.

### ASSAYS AND ANALYSIS OF THE ELICITOR ACTIVITY

In a first stage of the research which lead to the subject matter of the present invention, at the Biochemistry and Molecular Biology Lab of the International Center of Physics (Centro Internacional de Física (CIF)), studies at the lab related with the capability of several sterile preparations of the fungus *Fusarium ssp* were developed, in order to induce (elicit) SIR at different times, in various models (tomato, naranjilla, carnation and two native varieties of the genus *Solanum Lam,* lulo de perro and lulo llanero), obtaining promissory results.

Subsequently, a research phase in the field, using potato crops, was started quantifying the expression of some biochemical markers of resistance as parameters of SIR, simultaneously with the determination of contamination indexes (microbiological, biochemical and edaphic parameters) in soils. Field results confirm the laboratory observations. The induction of systemic resistance with the product is effective for the control of fungal diseases (data non published).

Some assays carried out with the elicitor of the present invention are described afterwards. The assays of the examples given immediately and described in figures 1 through 6 illustrate some of the effects of the invention over the plants, without limiting its scope. The behavior of some biochemical markers of systemic resistance is showed by quantifying mechanism of the conferred resistance to the plants once they have received the treatment with the product. The illustrated markers of resistance are:
■ Callose contents, a β-1,3 glucan which accumulates in lamellas, sieve tubes and cell walls of the plants inhibiting the penetration of fungus, bacteria, and virus.
■ β-1,3 glucanase activity, a hydrolytic enzyme which degrades the cell walls of fungi and bacteria.
■ Peroxidase activity, an enzyme which stimulates the accumulation of lignin and hydroxyproline rich glycoproteins (HPRG) in the plant walls, conforming barriers against fungal and bacteria penetration.

### Example 4

### Determination of Callose contents

The purpose of the present assay was to determine the effect of the elicitor of the present invention over the callose contents in leaves and roots of three species of the genus *Solanum Lam (S. quitoense* (susceptible to *Fusarium*), *S. marginatum* (resistant to *Fusarium)* and *S. pseudolulo* (susceptible to *Fusarium*)). In order to analyze this, seeds of the three species were sown under sterile conditions (in vitro), and the leaves were treated with 20 µl of the elicitor together with sodium carbonate (agricultural composition), 20 days post-germination. Samples (5 replicates) of leaves and roots from treated plants and controls were taken at 0, 20, 40 and 60 days post treatment. Polysaccharides extraction was done and callose contents quantified following the process described by Kohle et al. (Kohle, H., Jeblick, W., Poten, F., Blaschek, W., Kauss, H., 1985. Chitosan elicited callose synthesis in soybean cells as a Ca²⁺ dependent process. Plant Physiol. 77: 544-551.) and Kauss (Kauss H., 1992, Callose and callose synthase, In: Gurr, S., McPherson M.J. and Bowels, D.J. (Eds). Molecular Plant Pathology. A practical approach. Vol II. IRL Press. Pages 1-8). Said process consists of precipitation of polysaccharides with ethanol 95%, solubilization in NaOH 2N heating and centrifugation, taking the supernatants. The quantity of β-1,3 glucan was determined by fluorometry using the affinity of sirofluor of aniline blue for β-1,3 bonds. As it can be observed in Figure No. 1, the leaves of the plants of *S. quitoense* and *S. pseudolulo* reach and exceed the constitutive and induced levels of callose of *S. marginatum* 20 days post treatment, those of *S. quitoense* keep high, while those of *S. pseudolulo* decrease under the constitutive levels of S. *marginatum.*

In Figure No. 2, the callose behavior in roots is observed, wherein the accumulation of this polysaccharide is slower than in the leaves. However, *S. quitoense* reaches the levels induced in *S. marginatum* at the 60th day post treatment while *S. pseudolulo* reaches the constitutive levels of *S. marginatum.*

Observing both figures (1 and 2), it can be concluded that:
■ The elicitor of the present invention induces the accumulation of callose in the three species in a systemic (in new leaves and roots) and gradual way.
■ The accumulation of this polysaccharide, induced by the product, is similar or superior in the plants susceptible with respect to the resistant one, 20 days post treatment in the leaves.
■ The duration of the effect of the treatment with the product, regarding the accumulation of callose in the leaves and the roots, is at least 60 days with just one application.

### Example 5

### Determination, of β-1,3 glucanase activity

The purpose of the present assay was to determine the effect of the elicitor, of the present invention, over the β-1,3 glucanase activity of leaves and roots from the three species of the genus *Solanum Lam (S. quitoense* (susceptible to *Fusarium*), *S. marginatum* (resistant to *Fusarium*) and *S. pseudolulo* (susceptible to *Fusarium*)). In order to carry out, seeds of the three species were sown under sterile conditions (in vitro), and they were treated with 20 µl of the product, 20 days post-germination. Samples (5 replicates) of leaves and roots from the treated plants and from the control were taken 0, 20, 40 and 60 days post treatment. A total proteins extraction was carried out and β-1,3 glucanase activity was quantified for the treated leaves or roots and controls extracts. The quantification of the β-1,3 glucanase activity followed the methodology described by De Tapia et al. (De Tapia, M., Bergmann, P., Awade, A., & Burkward, C., 1986 Analysis of acid extractable bean leaf protein induced by mercuric chloride treatment and alfalfa mosaic virus. Partial purification and characterization. Plant Science, 45: 167-177). Said methodology consists of taking an aliquot from the crude extract together with a solution of the specific substrate (oarweed laminarin from *Laminaria digitata*), incubating at 37 °C for 1 hour stopping the reaction with ice and subsequently, measuring the production of reducing sugars from oarweed laminarin using the methodology proposed by Nelson (Nelson, N. 1944. A photometric adaptation of the Somogyi method for the determination of sugars. Journal of Biological Chemistry. 153: 257-262) and Somogyi (Somogyi, M. 1952. Notes on sugar determination. Journal of Biological Chemistry. 195: 19-23).

In Figure No. 3, the changes in the β-1,3 glucanase activity are observed in the leaves of the three species. *S. quitoense* reaches the β-1,3 glucanase activity levels induced in *S. marginatum* 20 days post treatment, decreasing to the constitutive levels of *S. marginatum* in the 40th day, and increasing again in the 60th day. *S. pseudolulo* has a continuous increase in its glucanase activity, reaching at the 60th day post treatment the constitutive levels of *S. marginatum.*

The β-1,3 glucanase activities of the roots of the three species are indicated in Figure No. 4. It is observed that *S. quitoense* as well as *S. pseudolulo* accumulate this enzyme in their roots over the constitutive values of *S. marginatum* starting at the 20th day post treatment, being maintained along the 60 days of the assay.

Observing both figures (3 and 4), it can be concluded that:
■ The elicitor of the present invention induces the β-1,3 glucanase activity in the three species in a systemic (in new leaves and roots) and gradual way.
■ The activity of this enzyme, induced by the elicitor of the present invention, is similar in the susceptible plants to the resistant one by the 20th day post treatment in the leaves and in the roots.
■ The duration of the effect of the treatment with the elicitor of the present invention, regarding the glucanase activity in the leaves and roots, is at least 60 days, with just one application.

### Example 6

### Determination of the β-1,3 glucanase activity and of the peroxidase activity in potato plants

The purpose of the present assay was to determine the effect of the elicitor of the present invention over the β-1,3 glucanase activity and the peroxidase activity of the leaves and roots of potato (*Solanum tuberosum*) of the variety (R-12) susceptible to *Phytophtora infestans* compared to that of an absolute witness (control) and to a treatment with chemical fungicides for the control of this disease. In order to develope this, 900 plants were field cultivated (Sisga municipality), the plot was divided in nine areas (100 plants per area, 3 replicates per treatment). Samples (3 per treatment per replica) from leaves and roots were taken starting germination (30 days after the sowing) and monthly thereon. Total proteins from leaves and roots were quantified as well as the β-1,3 glucanase activity and the peroxidase activity of leaves and roots treated with the elicitor of the present invention, with the pesticide and the control. β-1,3 glucanase activity was quantified following the methodology described by De Tapia et al. and by Nelson (1944) and Somgyi (1952), established in example 5.

Changes in β-1,3 glucanase activity of the plant leaves treated with the elicitor of the present invention, pesticide and control are shown in Figure No. 5 along the 150 days of cultivation. β-1,3 glucanase activities of the roots of the three species are indicated in Figure No. 6.

It is observed that the potato plants treated with the elicitor of the present invention accumulate this enzyme in their roots with values 70% over those of the control and pesticide treatments, which are maintained along the cultivation period.

The quantification of peroxidase activity was carried out following the methodology described by Ampomah (Ampomah, S.A., Ye, X.S., Kuc, J., 1995. Detection of several enzymatic activities in leaf prints of cucumber plants. Physiol. Mol. Plant Pathol. 42: 441-454). Said methodology consists of a spectrophotometric pursuit of the kinetics reaction of an aliquot from the crude extract of total proteins, together with hydrogen peroxide and guaiacol.

The peroxidase activity of leaves and roots from potato are showed in Figures 7 and 8, respectively, these include the three treatments along 150 days of monitory. An increase of the peroxidase activity of the plants submitted to the elicitor of the present invention, was observed in contrast to the control and pesticide treatments.

Summarizing the behavior of the β-1,3 glucanase activity and the peroxidase activity of the leaves and roots of potato plants treated with the elicitor of the present invention, it can be concluded that:
■ The defense capability of plants against fungi, bacteria and virus increases, with the synthesis of antifungal and antibacterial compounds.
■ The duration of the response goes from several weeks to months.
■ The elicitor of the present invention stimulates defense responses, systemic (leaves and roots), which are not stimulated by chemical pesticides.

The embodiments of the present invention can, of course, be carried out in other ways different to those described herein, without moving away from the spirit and scope of the invention. The present embodiments should be considered in all aspects as illustrative and non restrictive. All changes and equivalents should also be comprised within the specification of the present invention.

## Claims

1. A process for preparing an elicitor of systemic resistance from *Fusarium oxysporum,* **characterized by** comprising the steps of:
(i) revirulentation of the fungus *Fusarium oxysporum* in a host and culturing said fungus;
(ii) sequentially- extracting the fungal components by means of the steps of:
a) filtration and washing the fungal mycelium with distilled water or with aqueous solutions;
b) breakage through freezing the isolated mycelium between -40 °C and -170 °C, followed by powdering;
c) extraction of the powder obtained in b) using a buffer in a pH range between 4 and 7, for its subsequent- sonication by ultrasound, sterilization by heat and centrifugation of the resulting suspension; being the supernatant fraction A;
d) extraction of the centrifugation residual of the suspension using an alcoholic solvent and subsequent storage at a temperature from 0 to 10 °C between 20 and 200 hours, elimination of the alcoholic solvent, and resuspension in a basic solution with a pH from 12 to 14 with incubation at a temperature from 70 to 100 °C for at least 0.5 hours. The obtained mixture is adjusted to a pH from 5,0 to 7,0 with an acid solution and then, centrifuged, being the supernatant fraction B;
(iii) mixture of fractions A and B obtained in step ii), being fraction A between 10 and 50% of the mixture and fraction B between 90 and 50% of the mixture.

2. The process for preparing an elicitor of systemic resistance from *Fusarium oxysporum* according with claim 1, wherein the breakage through freezing of the fungus *Fusarium oxysporum* is carried out with liquid nitrogen or other liquefied gas at a temperature from -40 °C to -170 °C.

3. The process for preparing an elicitor of systemic resistance from *Fusarium oxysporum* according with any one of claims 1 and 2, wherein the buffer used in step (ii) is selected from the group consisting of sodium phosphate, citrate or ascorbate.

4. The process for preparing an elicitor of systemic resistance from *Fusarium oxysporum* according with any one of the preceding claims, wherein the sterilization by heat carried out in step (ii) is made in autoclave.

5. The process for preparing an elicitor of systemic resistance from *Fusarium oxysporum* according with any one of the preceding claims, wherein the alcoholic solvent used in step (ii) is ethanol.

6. The process for preparing an elicitor of systemic resistance from *Fusarium oxysporum* according with any one of the preceding claims, wherein the basic solution used in step (ii) is a solution of NaOH with a concentration from 0.01 to 2N

7. The process for preparing an elicitor of systemic resistance from *Fusarium oxysporum* according with any one of the preceding claims, wherein the acid solution used in step (ii)(d) is a solution of HCl with a concentration from 6 to 12 N.

8. The process for preparing an elicitor of systemic resistance from *Fusarium oxysporum* according with any one of the preceding claims, wherein in the mixture of step (iii), fraction A is a 17% of the mixture and fraction B is a 83% of the mixture.

9. An elicitor of systemic resistance from *Fusarium oxysporum,* **characterized by** comprising a mixture of a fraction A and a fraction B obtained by means of the process of claim 1.

10. The elicitor of systemic resistance from *Fusarium oxysporum* according to claim 9, wherein fraction A, obtained in the process of claim 1, contains proteins, among which from 70 to 95% are glycoproteins.

11. The elicitor of systemic resistance from *Fusarium oxysporum* according to claim 10, wherein from 15 to 20% of said glycoproteins contain mannose and arabinose residues, and from 80% to 85% contain galactose residues.

12. The elicitor of systemic resistance from *Fusarium oxysporum* according, to any one of claims 10 or 11, wherein said glycoproteins have anionic characteristics and they are in a molecular weight range from 6.800 Da (1,122 x 10⁻²⁰ g) to 76.000 Da (1,254 x 10⁻¹⁹ g).

13. The elicitor of systemic resistance from *Fusarium oxysporum* according to any one of claims 9 to 12, wherein fraction B is composed by proteins and polysaccharides from the cell wall of the fungus *Fusarium oxysporum*.

14. The elicitor of systemic resistance from *Fusarium oxysporum* according to claim 13, wherein 60% of said proteins are mannoproteins having anionic characteristics and they are in a molecular weight range from 10.000 Da (1,65 x 10⁻²⁰ g) and 65.000 Da (1,0725 x 10⁻¹⁹ g).

15. The elicitor of systemic resistance from *Fusarium oxysporum* according to any one of claims 13 or 14, wherein from 55 to 70% of said polysaccharides contain glucans, out of which from 15 to 25% are β-1,3 bonds, and from 0.1 to 0.9% are N-acetyl glucosamine residues, and from 8 to 13% of said polysaccharides contain complex hemicelluloses of the xyloglucans type and reducing sugars.

16. The use of the elicitor of systemic resistance from *Fusarium oxysporum* according to any one of claims 9 to 15, for the manufacture of an agricultural composition which accelerates the defense metabolism of the plants.

17. An agricultural composition, **characterized by** comprising the elicitor of systemic resistance from *Fusarium oxysporum* according to any one of claims 9 to 16 together with an acceptable agricultural carrier.

18. The agricultural composition according to claim 17, wherein the agricultural carrier should not contain ionic or non ionic detergents.

19. The agricultural composition according to any one of claims 17 or 18, wherein the acceptable agricultural carrier is sodium carbonate with a concentration from 30 to 60 mM.

20. The agricultural composition according to any one of claims 17 to 19, **characterized by** being applied by spraying.
